# EUROPÄISCHE PATENTSCHRIFT

(11) **EP 0 713 861 B1**
(45) Veröffentlichungstag und Bekanntmachung des Hinweises auf die Patenterteilung: **03.02.1999**
(21) Anmeldenummer: 95118129.6
(22) Anmeldetag: 17.11.1995
(51) Int. Cl.: C07C 249/02, C07C 251/16

(54) **Verfahren zur Herstellung von Benzophenonimin**
Process for the preparation of benzophenone imine
Procédé pour la préparation de benzophénone imine

(30) Priorität: 26.11.1994 DE 44421389
(43) Veröffentlichungstag der Anmeldung: 29.05.1996
(73) Patentinhaber: BASF AKTIENGESELLSCHAFT, 67056 Ludwigshafen (DE)
(72) Erfinder: Voit, Guido, Dr., D-69198 Schriesheim (DE); Holderbaum, Martin, Dr., D-67065 Ludwigshafen (DE); Witzel, Tom, Dr., D-67069 Ludwigshafen (DE); Aumüller, Alexander, Dr., D-67435 Neustadt (DE)

(56) Entgegenhaltungen:
- FR-A- 2 364 201
- GB-A- 1 280 551
- US-A- 2 533 723

## Beschreibung

Die vorliegende Erfindung betrifft ein Verfahren zur Herstellung von Benzophenoniminen aus Benzophenonen in flüssigem Ammoniak.

Bei der Direktumsetzung von Benzophenon in Schmelze oder Lösung mit gasförmigem oder flüssigem Ammoniak müssen Eisen(III)chlorid (JP-A-61/030563, Ausbeute: 57%), organische Säuren wie z.B. Benzoesäure (US-A-4 130 586, Ausbeuten: 18 bis 65%) oder Ammoniumchlorid (Synthetic Communications 18 (1988) 1501 bis 1511; Ausbeute 94%) als Katalysator zugesetzt werden. Die Nachteile dieser Verfahren liegen in zum Teil niedrigen Ausbeuten aber vor allem in einer aufwendigen Abtrennung des Katalysators bzw. in einem Zwangsanfall an Salz.

Unter drastischen Bedingungen (400°C) läßt sich Benzophenon in Gasphase heterogenkatalysiert an Thoriumoxid [Nippon Kagaku Kaishi (1973) 1392 bis 1396 oder Compt. Rend. 169 (1919) 237 bis 239] oder Thoriumoxid/Siliciumoxid [Nippon Kagaku Kaishi (1974) 2216 bis 2218] umsetzen. Nachteilig an diesen Verfahren ist die aufwendige Technik (hohe Temperaturen, hohes Vakuum) und ein hoher Anteil an Zersetzungsprodukten.

Die heterogenkatalysierte Umsetzung von Benzophenon in flüssiger Phase mit gasförmigem Ammoniak an Ionentauschem ist aus Chemistry Letters (1976) 205 bis 206 bekannt. Die Ausbeuten liegen jedoch unbefriedigend niedrig bei 10%.

Aus der US-A-4 083 869 ist ein Verfahren zur Herstellung von Benzophenonimin bekannt, bei dem Benzophenon in flüssiger Phase, vorzugsweise in einem organischen Lösungsmittel, an Oxiden von Metallen der 2. bis 5. Periode der III. bis V. Gruppe wie z.B. Titanoxid oder Aluminiumoxid bei Temperaturen zwischen 150 bis 250°C und Drücken zwischen 1 und 50 Atmosphären mit gasförmigem bzw. überkritischem Ammoniak umgesetzt wird. Die Ausbeuten von max. 60% sind jedoch unzureichend.

Der vorliegenden Erfindung lag daher die Aufgabe zugrunde, den zuvor genannten Nachteilen abzuhelfen.

Demgemäß wurde ein Verfahren zur Herstellung von Benzophenoniminen der allgemeinen Formel I 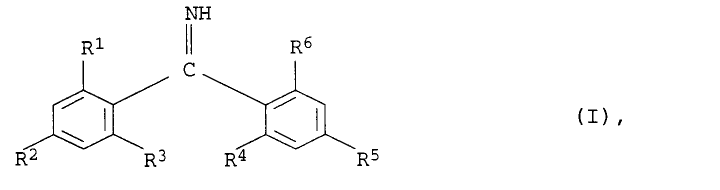 in der R¹ bis R⁶ Wasserstoff, C₁- bis C₄-Alkoxy, C₁- bis C₂-Alkylamin und C₂- bis C₄-Dialkylamin bedeuten, gefunden, welches dadurch gekennzeichnet ist, daß man Benzophenone der allgemeinen Formel II 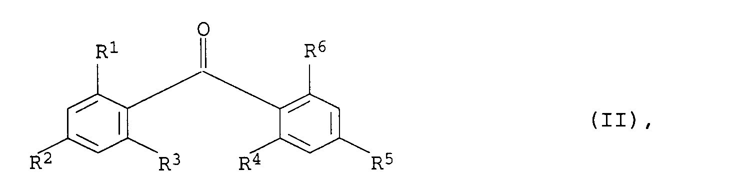 in der R¹ bis R⁶ die oben genannten Bedeutungen haben, in flüssigem Ammoniak in Gegenwart von Oxiden der Elemente Bor, Aluminium, Gallium, Indium, Silicium, Germanium, Zinn, Blei, Phosphor, Arsen, Antimon, Wismut, Scandium, Yttrium, Titan, Zirkon, Vanadin, Niob, Tantal oder deren Gemische bei Temperaturen von 80 bis 140°C und Drücken von 150 bis 250 bar umsetzt.

Das erfindungsgemäße Verfahren wird wie folgt durchgeführt:

Das Verfahren kann entweder diskontinuierlich im Rührautoklaven oder kontinuierlich im Rohrreaktor, bevorzugt kontinuierlich im Rohrreaktor, bei Temperaturen von 80 bis 140°C, besonders bevorzugt von 120 bis 140°C und Drücken von 150 bis 250 bar, besonders bevorzugt von 180 bis 220 bar, durchgeführt werden.

Als Katalysatoren kommen Oxide oder Mischoxide der Elemente Bor, Aluminium, Gallium, Indium, Silicium, Germanium, Zinn, Blei, Phosphor, Arsen, Antimon, Wismut, Scandium, Yttrium, Titan, Zirkon, Vanadin, Niob, Tantal, bevorzugt Oxide oder Mischoxide der Elemente Bor, Aluminium, Gallium, Silicium, Zinn, Blei, Antimon, Wismut, Titan, Zirkon, Vanadin, Niob, besonders bevorzugt Oxide oder Mischoxide der Elemente Aluminium, Silicium, Titan, Zirkon, Vanadin, insbesondere Titanoxide in Betracht.

Die Katalysatoren können in Form von Pulver (Rührautoklav) oder in Form von Tabletten oder Strängen (Rohrreaktor) eingesetzt werden.

Die Abtrennung des Reaktionswassers während oder nach der Reaktion ist nicht notwendig.

Bei der kontinuierlichen Fahrweise wird in der Regel eine Belastung von 0.1 bis 0.6 kg Benzophenon pro Liter Katalysator und Stunde eingestellt, bevorzugt 0.2 bis 0.4 kg/lh.

Benzophenon kann in Schmelze oder in Lösung, bevorzugt in Schmelze eingesetzt werden.

Der Ammoniak wird in flüssiger Form eingesetzt. Bevorzugt werden 1 bis 10 kg Ammoniak pro kg Benzophenon eingesetzt, besonders bevorzugt 3 bis 6 kg/kg.

Benzophenonimin dient als Vorprodukt der Herstellung von Lichtschutzmitteln (z.B. 2-Cyano-3,3-diphenylacrylsäure-ethylester) [Bull. Chem. Soc. Fr. (1963) 1576-1583].

Die Substituenten R¹ bis R⁶ in den Verbindungen I und II haben folgende Bedeutungen:
R¹, R², R³, R⁴, R⁵, R⁶ unabhängig voneinander
   - Wasserstoff,
   - C₁- bis C₄-Alkoxy wie Methoxy, Ethoxy, n-Propoxy, iso-Propoxy, n-Butoxy, iso-Butoxy, sec.-Butoxy und tert.-Butoxy, bevorzugt Methoxy, Ethoxy, n-Propoxy und n-Butoxy, besonders bevorzugt Methoxy und Ethoxy,
   - C₁- bis C₂-Alkylamin wie Methylamin und Ethylamin, bevorzugt Methylamin,
   - C₂- bis C₄-Dialkylamin wie Dimethylamin und Diethylamin, bevorzugt Dimethylamin.

Besonders bevorzugt ist die Verbindung, in der R¹ bis R⁶ Wasserstoff bedeutet.

### Beispiele

### Beispiel 1

In einem 250 ml Rührautoklaven wurden 15 g Benzophenon in 125 ml Ammoniak zusammen mit 2 g Titanoxid-Pulver 5 h bei 200 bar und 130°C gerührt. Nach Abdestillieren des Ammoniaks und Abtrennen des

Katalysators wurde der zweiphasige Austrag mit Dimethylformamid homogenisiert und mittels GC untersucht:
- Umsatz:: 95%
- Selektivität:: 99%

### Beispiel 2

Durch einen Rohrreaktor - gefüllt mit 300 ml Titanoxid (3 mm Stränge) - wurden 180 g Benzophenon/h und 720 g Ammoniak/h bei 200 bar und 130°C gefahren. Nach Abdestillieren des Ammoniaks wurde der zweiphasige Austrag mit Dimethylformamid homogenisiert und mittels GC untersucht:
- Umsatz:: 95%
- Selektivität:: 99%

### Beispiel 3

Durch einen Rohrreaktor - gefüllt mit 300 ml Titanoxid (3 mm Stränge) - wurden 60 g Benzophenon/h und 720 g Ammoniak/h bei 200 bar und 130°C gefahren. Nach Abdestillieren des Ammoniaks wurde der zweiphasige Austrag mit Dimethylformamid homogenisiert und mittels GC untersucht:
- Umsatz:: 98%
- Selektivität:: 99%

### Beispiel 4

Durch einen Rohrreaktor - gefüllt mit 60 ml Titanoxid (3 mm Stränge) - wurden in der Stunde 24 g einer 25%igen methanolischen Benzophenonlösung und 36 g Ammoniak bei 120 bar und 130°C gefahren. Nach Abdestillieren des Ammoniaks wurde der Austrag mittels GC untersucht:
- Umsatz:: 91%
- Selektivität:: 99%

### Beispiel 5

Durch einen Rohrreaktor - gefüllt mit 60 ml Aluminiumoxid (3 mm Stränge) - wurden in der Stunde 24 g einer 25%igen methanolischen Benzophenonlösung und 36 g Ammoniak bei 120 bar und 130°C gefahren. Nach Abdestillieren des Ammoniaks wurde der Austrag mittels GC untersucht:
- Umsatz:: 58%
- Selektivität:: 99%

## Patentansprüche

1. Verfahren zur Herstellung von Benzophenoniminen der allgemeinen Formel I 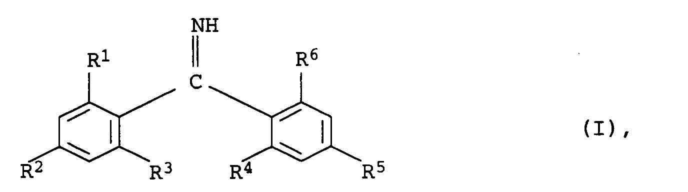 in der R¹ bis R⁶ Wasserstoff, C₁- bis C₄-Alkoxy, C₁- bis C₂-Alkylamin und C₂- bis C₄-Dialkylamin bedeuten, dadurch gekennzeichnet, daß man Benzophenone der allgemeinen Formel II 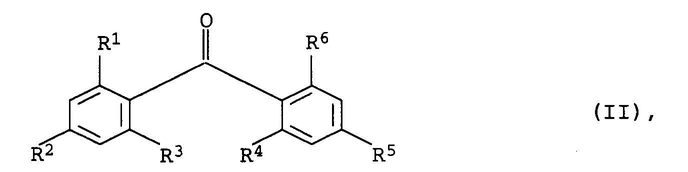 in der R¹ bis R⁶ die oben genannten Bedeutungen haben, in flüssigem Ammoniak in Gegenwart von Oxiden der Elemente Bor, Aluminium, Gallium, Indium, Silicium, Germanium, Zinn, Blei, Phosphor, Arsen, Antimon, Wismut, Scandium, Yttrium, Titan, Zirkon, Vanadin, Niob, Tantal oder deren Gemische bei Temperaturen von 80 bis 140°C und Drücken von 150 bis 250 bar umsetzt.

2. Verfahren zur Herstellung von Benzophenoniminen nach Anspruch 1, dadurch gekennzeichnet, daß R¹ bis R⁶ Wasserstoff bedeuten.

3. Verfahren zur Herstellung von Benzophenoniminen nach Anspruch 1, dadurch gekennzeichnet, daß man als Oxide solche der Elemente Bor, Aluminium, Gallium, Silicium, Zinn, Blei, Antimon, Wismut, Titan, Zirkon, Vanadin, Niob oder deren Gemische einsetzt.

4. Verfahren zur Herstellung von Benzophenonimin nach Anspruch 1, dadurch gekennzeichnet, daß man als Oxide solche der Elemente Aluminium, Silicium, Titan, Zirkon, Vanadin oder deren Gemische einsetzt.

5. Verfahren zur Herstellung von Benzophenonimin nach Anspruch 1, dadurch gekennzeichnet, daß man als Oxide Titanoxide einsetzt.

6. Verfahren zur Herstellung von Benzophenonimin nach Anspruch 1, dadurch gekennzeichnet, daß man die Umsetzung bei Temperaturen von 120 bis 140°C und Drücken von 180 bis 220 bar durchführt.

## Claims

1. A process for preparing benzophenone imines of the general formula I 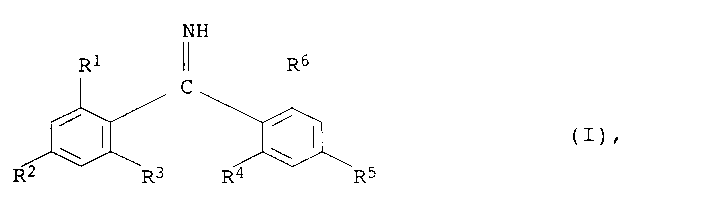 where R¹ to R⁶ are hydrogen, C₁- to C₄-alkoxy, C₁- to C₂-alkylamine or C₂- to C₄-dialkylamine, which comprises reacting benzophenones of the general formula II 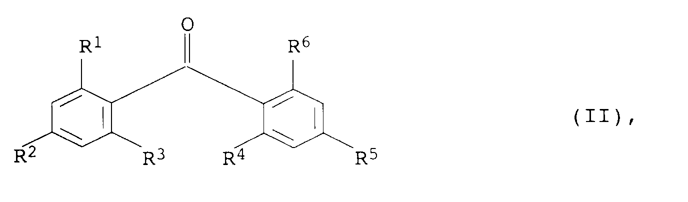 where R¹ to R⁶ have the abovementioned meanings, in liquid ammonia in the presence of oxides of the elements boron, aluminum, gallium, indium, silicon, germanium, tin, lead, phosphorus, arsenic, antimony, bismuth, scandium, yttrium, titanium, zirconium, vanadium, niobium, tantalum or mixtures thereof at from 80 to 140°C and from 150 to 250 bar.

2. A process for preparing benzophenone imines as claimed in claim 1, wherein R¹ to R⁶ are hydrogen.

3. A process for preparing benzophenone imines as claimed in claim 1, wherein the oxides empoloyed are those of the elements boron, aluminum, gallium, silicon, tin, lead, antimony, bismuth, titanium, zirconium, vanadium, niobium or mixtures thereof.

4. A process for preparing benzophenone imines as claimed in claim 1, wherein the oxides employed are those of the elements aluminum, silicon, titanium, zirconium, vanadium or mixtures thereof.

5. A process for preparing benzophenone imines as claimed in claim 1, wherein the oxides employed are titanium oxides.

6. A process for preparing benzophenone imines as claimed in claim 1, wherein the reaction is carried out at from 120 to 140°C and from 180 to 220 bar.

## Revendications

1. Procédé de préparation de benzophénonimines de la formule générale I : 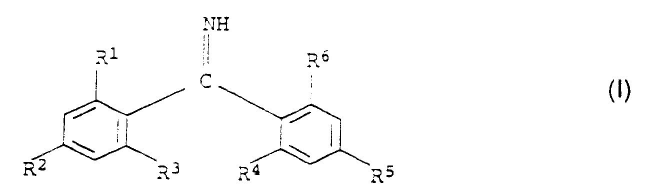 dans laquelle les symboles R¹ à R⁶ représentent chacun individuellement un atome d'hydrogène, un radical alcoxy en C₁ à C₄, alkylamine en C₁ ou C₂ et dialkylamine en C₂ à C₄, caractérisé en ce que l'on fait réagir des benzophénones de la formule générale II : 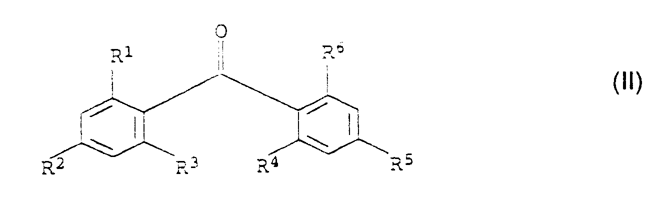 dans laquelle les symboles R¹ et R⁶ ont les significations qui leur ont été attribuées ci-dessus, dans de l'ammoniac liquide, en présence d'oxydes des éléments bore, aluminium, gallium, indium, silicium, germanium, étain, plomb, phosphore, arsenic, antimoine, bismuth, scandium, yttrium, titane, zirconium, vanadium, niobium, tantale ou leurs mélanges à des températures qui varient de 80 à 140°C et sous des pressions qui fluctuent de 150 à 250 bars.

2. Procédé de préparation de benzophénonimines suivant la revendication 1, caractérisé en ce que les symboles R¹ à R⁶ représentent des atomes d'hydrogène.

3. Procédé de préparation de benzophénonimines suivant la revendication 1, caractérisé en ce que l'on utilise, à titre d'oxydes, ceux des éléments bore, aluminium, gallium, silicium, étain, plomb, antimoine, bismuth, titane, zirconium, vanadium, niobium ou leurs mélanges.

4. Procédé de préparation de benzophénonimines suivant la revendication 1, caractérisé en ce que l'on utilise, à titre d'oxydes, ceux des éléments aluminium, silicium, titane, zirconium, vanadium ou leurs mélanges.

5. Procédé de préparation de benzophénonimines suivant la revendication 1, caractérisé en ce que l'on utilise, à titre d'oxydes, des oxydes du titane.

6. Procédé de préparation de benzophénonimines suivant la revendication 1, caractérisé en ce que l'on entreprend la réaction à des températures de 120 à 140°C et sous des pressions de 180 à 220 bars.
